# EUROPÄISCHE PATENTSCHRIFT

(11) **EP 0 839 538 B1**
(45) Veröffentlichungstag und Bekanntmachung des Hinweises auf die Patenterteilung: **16.04.2003**
(21) Anmeldenummer: 97118212.6
(22) Anmeldetag: 21.10.1997
(51) Int. Cl.: A61L 9/16

(54) **Luftreinigungsgerät**
Air purifying apparatus
Appareil pour la purification de l'air

(30) Priorität: 01.11.1996 DE 19645096
(43) Veröffentlichungstag der Anmeldung: 06.05.1998
(73) Patentinhaber: Clinix GmbH, 9400 Rorschach (CH)
(72) Erfinder: Hammes, Frank, 9326 Horn (CH)
(74) Vertreter: Riebling, Peter, Dr.-Ing.

(56) Entgegenhaltungen:
- EP-A- 0 123 824
- DE-A- 3 205 454
- DE-A- 3 424 921
- DE-U- 7 506 026
- US-A- 4 742 764

## Beschreibung

Die Erfindung betrifft ein Luftreinigungsgerät nach dem Oberbegriff des Patentanspruchs 1. Derartige Luftreinigungsgeräte sind in vielfältigen Ausführungsformen bekannt geworden. Sie bestehen im wesentlichen aus einem Ansaugteil, einem Ventilatorgehäuse, in dem ein oder mehrere Ventilatoren angeordnet sind und aus einem oder mehreren Luftfiltern, die vor oder nach dem Ventilator angeordnet sind, um die Luft von einer bestimmten Ansaugöffnung anzusaugen, durch das Ventilatorgehäuse hindurchzupressen und durch die Luftfilter hindurchzuführen, um dort den Staub oder andere Schwebstoffe abzuscheiden.

Eine solche Vorrichtung ist aus der EP 0 123 824 zu entnehmen, die in etwa kübelförmig aufgebaut ist und in ihrem oberen Bereich die Ansaugöffnung und in ihrem unteren Bereich die Auslassöffnung ausgebildet hat. Sie ist somit in der Form von übereinander angeordneten Einheiten zusammengebaut, wobei diese im Wesentlichen aus einem oberen und einem unteren Teil bestehen. Das Oberteil ist somit einfach auf das Unterteil aufgestülpt.

Nachteil der bisher bekannten Luftreinigungsgeräte, die mit dem Trockenfilterprinzip arbeiten, ist jedoch die schlechte Auswechselbarkeit der Filter.

### Hierunter wird folgendes verstanden:

Bei den bisher bekannten Geräten sind mehrere verschiedene Filtermedien in einem einzigen Filterelement zusammengefasst, was mit dem Nachteil verbunden ist, daß das Filterelement nur insgesamt ausgewechselt werden kann, was mit relativ hohen Kosten verbunden ist und außerdem zumeist unnötig ist, weil nur einer der verschiedenen, im Element zusammengefassten Filtertypen verschmutzt und auswechslungsbedürftig ist, während die anderen noch nicht auswechslungsbedürftig sind. Daraus ergibt sich der Nachteil, daß also die einzelnen Filter des Luftreinigungsgeräts in der Regel nicht einzeln auswechselbar sind, sondern nur insgesamt als Filterelement auswechselbar sind.

Ein weiterer Nachteil der bekannten Luftreinigungsgeräte ist, daß die Filter nicht im Gehäuse des Luftreinigungsgeräts an unterschiedlichen Stellen angeordnet werden können. Beispielsweise ist es manchmal erwünscht, den Grobstaubfilter möglichst in der Nähe der Ansaugöffnung des Geräts anzuordnen, um dort eine maximale Staubmenge von Grobstaub zur Abscheidung zu bringen, und auch andere Teile des Geräts vor diesem Grobstaub zu schützen. Nun ist es bei den bekannten Geräten nicht möglich, z.B. dem Grobstaubfilter mehr als einen nachgeschalteter Feinstaubfilter zuzuordnen, weil die Reihenfolge der Filter und die Anzahl der Filter in den bekannten Luftreinigungsgeräten genau vorgegeben ist und durch die Gehäuseform und die Gehäuseanordnung bestimmt ist.

Ein weiterer Nachteil der bekannten Luftreinigungsgeräte ist zum Dritten, daß die Filter als solches nicht schnell auswechselbar sind, d.h. es bedarf beträchtlicher Demontagearbeiten, um derartige Filter aus dem Gehäuse herauszuholen. Hierbei ist es bekannt, das Gehäuse über Kniehebelverschlüsse oder über andere Spannverschlüsse zu verschließen, die sehr mühevoll zu öffnen sind.

Ein weiterer, vierter Nachteil der bekannten Luftreinigungsgeräte ist, daß in der Regel bei den bekannten Geräten der Motorteil als letztes direkt vor dem Ausströmteil angeordnet ist. Dies ist mit dem Nachteil verbunden, daß von dem Motor selbst Fremdstoffe während des Betriebes abgegeben werden, z.B. Partikel aufgrund der Reibung in den Lagern, Kunststoffteilchen vom Ventilatorflügel und anderes mehr, so daß der Motor selbst aufgrund seiner Tätigkeit Partikel in den zu reinigenden Raum einträgt, was dazu führt, daß eine Reinraumatmosphäre nicht erreichbar ist.

Der Erfindung liegt deshalb die Aufgabe zugrunde, ein Luftreinigungsgerät der eingangs genannten Art so weiterzubilden, daß auf einfache Weise einzelne Filter in dem Luftreinigungsgerät schnell auswechselbar angeordnet sind, und daß die Reihenfolge der Filter und der verschiedenen anderen Funktionsteile des Luftreinigunggsgeräts relativ beliebig variiert werden kann, und diese einfach miteinander verbunden und gesichert werden können.

Zur Lösung der gestellten Aufgabe ist die Erfindung durch die technische Lehre des Anspruchs 1 gekennzeichnet.

Wesentliches Merkmal der Erfindung ist, daß nun erfindungsgemäss die einzelnen Funktionsteile des Luftreinigungsgeräts modulartig ausgebildet sind, und daß die Module übereinander gestapelt (stapelbar) einen turmartigen Aufbau ergeben, wobei dieser Aufbau durch mindestens einen, außen liegenden Schwenkhebel zusammengespannt wird.

Mit der gegebenen technischen Lehre der Erfindung ergibt sich der wesentliche Vorteil, daß aufgrund der modulartigen Ausbildung der einzelnen Funktionsteile des Luftreinigungsgeräts und deren Übereinanderstapelbarkeit, nun eine Auswechselbarkeit der einzelnen Module untereinander ohne weiteres erfolgen kann. Zwar wird bei der vorliegenden Erfindung die Verwendung eines einzigen Schwenkhebels beansprucht, bevorzugt wird jedoch die Verwendung von zwei einen gegenseitigen Abstand zueinander einnehmenden und zueinander schwenkbar gelagerten Schwenkhebeln, welche den modulartigen und turmartigen Aufbau der einzelnen Funktionsteile von außen her übergreifen und zusammenspannen.

Damit ergibt sich der wesentliche Vorteil, daß beim Öffnen der Schwenkhebel gleichzeitig bei den Modulen die im Dichtsitz aufeinanderliegen, der Dichtsitz geöffnet wird und die Module voneinander abhebbar sind.

Damit ist es nun erstmals möglich, daß man jedes Modul in der Art einer Schublade in horizontaler Richtung aus dem turmartigen Aufbau des Geräts herauszieht und so schnell auswechselbar gestaltet.

Damit ist es nun erstmals möglich, daß z.B. mit wenigen Handgriffen (Öffnen der beiden Schwenkhebel) z.B. der Grobstaubfilter allein ausgetauschbar wird, weil dieser in einem einzigen Modul angeordnet ist.

Ferner ist es möglich, daß der oberhalb des Ventilatorgehäuses (welches ebenfalls in einem Modul eingebaut ist) sitzende Feinstaubfilter ebenfalls in der Art einer Schublade herausgezogen wird und so schnell auswechselbar ist. Auf diese Weise können auch andere Funktionsteile schnell, wie z.B.Ausströmteil, auswechselbar aus dem turmartigen Aufbau herausgezogen werden.

Damit ergibt sich der weitere Vorteil, daß auch die Länge der Schwenkhebel veränderbar ist, so daß bei der Verwendung von längeren Schwenkhebeln es nun möglich ist, auf den insgesamt gegebenen Aufbau, bei Abnahme des oberen Ausströmteils, ein weiteres modulartiges Filtergehäuse aufzusetzen, um andere Filtereigenschaften des Luftreinigungsgeräts zu erreichen.

Man kann also die Aufbauhöhe des modulartigen Gesamtgehäuses nach Belieben verändern und braucht für diesen Zweck nur andersartige Schwenkhebel mit andersartiger Länge zu verwenden und entsprechend in ihren Schwenklagern einzusetzen.

Das erfindungsgemässe Luftreinigungsgerät ist einfach und zuverlässig deckenmontierbar und kann als Punktabsaug-, Ventilations- oder Überdruckgerät oder als Reinluft-Reinigungsgerät eingesetzt werden.

Dabei wird zur Deckenmontage das Luftreinigungsgerät mit seinem Sockel in ein Deckenmontageblech eingerastet, wobei der einfache Filterwechsel auch bei dieser Geräte-Deckenmontage gewährleistet ist. Das zwischen Geräte-Sockel und Ansaugwannenteil angeordnete Filtermodul wird selbst bei voller Öffnung der Geräte-Schwenkhebel gehalten. Die zwischen dem Ausströmteil und dem Ventilatorgehäuseteil angeordneten Filtermodule sind so gehalten, daß bei leichter Öffnung der Schwenkarme die vom Ansatz gehaltenen Filtermodule problemlos aus dem Luftreinigungsgerät entnehmbar sind.

Zum Einsatz als Punktabsauggerät ist das Luftreinigungsgerät am Gerätesockel mit einem Anschlußteil für einen Punktsaugschlauch ausgebildet, über welchen Luft gezielt zum Gerät abgesaugt werden kann. Dieses Punktabsauggerät ist vielfältig, beispielsweise im Bereich des Arbeitsschutzes beim Absaugen von Schweissrauch, einsetzbar.

Der vorbeschriebene Punktabsauganschluß kann auch benutzt werden, um die Filterfunktion des Luftreinigungsgerätes mit einer Ventilationsfunktion zu ergänzen. Hierzu wird ein Kanal mit Aussenluftanschluß mit dem Ansauganschluß des Geräts verbunden. Die sauerstoffreiche Luft wird somit von aussen angesaugt und wird nachdem sie durch alle Filterstufen des Geräts geströmt ist, in den Umgebungsraum abgegeben.

Der modulare Aufbau des Luftreinigungsgeräts ermöglicht es auch, daß normalerweise eingesetzte Ausströmteil durch ein spezielles Ausströmteil zu ersetzen, welches den Ausstoßluftstrom mittels Trichter, Schlauch oder Kanal auf eine bestimmte Stelle richten kann. Dadurch ist eine gezielte Luftreinigungwirkung auf eine gewünschte Stelle möglich.

In der bevorzugten Ausgestaltung der Erfindung sind die Schwenkhebel so ausgebildet, daß sie mit oberen, etwa horizontal gerichteten Armen in zugeordnete Aufnahmen des oberen Moduls eingreifen, so daß von oben her alle Module gegen ein unteres, auf dem Boden stehendes Sockelteil gespannt werden. Die Schwenkhebel sitzen hierbei in entsprechenden Schwenklagern des Sockelteils ein und sind dort schwenkbar gehalten.

In einer weiteren, bevorzugten Ausgestaltung des Erfindungsgedankens ist es dann vorgesehen, daß die Schwenkhebel ihrerseits mit einwärts gerichteten Hebelarmen verbunden sind, die in der Art von einseitig gelagerten Hebeln an den Innenseiten des jeweiligen Schwenkhebels angeordnet sind. Jeder Hebelarm greift mit einem entsprechenden, am freien Ende fest angeordneten Ansatz in eine zugeordnete Rinne ein, welche Rinne sich im Übergangsbereich zwischen dem einen Modul und dem darüber lagernden Modul befindet. Die Bodenfläche der Rinne ist hierbei dem unteren Modul zugeordnet, während die Deckfläche der Rinne dem oberen Modul zugeordnet ist.

Beide Flächen sind als Abdrückflächen ausgebildet und laufen im Winkel (schräg zur Horizontalen aufwärts geneigt) in das jeweilige Modul hinein.

Wird nun der Schwenkhebel nach außen verschwenkt, dann drückt sich der am freien Ende des jeweiligen Hebelarmes angeordnete Ansatz an der oberen Abdrückfläche der Rinne ab, welche dem oberen Modul zugeordnet ist. Auf diese Weise kommt es zu einer in vertikaler Richtung gerichteten Trennbewegung zwischen dem oberen Modul und dem dazugehörenden, darunterliegenden, unteren Modul.

Die beiden Module werden also voneinander abgehoben und aus ihrem einander zugeordneten Dichtsitz entfernt.

Der vorher beschriebene Vorgang ist so zu verstehen, daß der beschriebene Hebelarm mit dem am freien Ende angeordneten Ansatz in jeweils zugeordnete, einander gegenüberliegende Rinnen im Bereich des Motorsteils und der damit verbundenen Ansaugwanne eingreift. Beide Module sind bevorzugt miteinander verschraubt, so daß beide Module zusammen angehoben werden, wenn die Schwenkarme nach außen verschwenkt werden. Dadurch wird aber das Motorteil und die damit verbundene Ansaugwanne von dem unteren modulartig ausgebildeten Filter abgehoben und gleichzeitig wird auch das oberhalb des Motorteils angeordnete weitere Filter ebenfalls angehoben und kann damit leicht von dem angehobenen Motorteil abgezogen werden.

Beim Einschwenken der Schwenkhebel in die Schließposition kommt es zu einer doppelten Verklemmung, denn einerseits greifen die Schwenkhebel mit etwa horizontal gerichteten Ansätzen auf das obere im Turm angeordnete Ausströmteil über, welches damit festgeklemmt wird und die entsprechende Gewichtsbelastung und die Klemmkraft werden auch alle anderen darunterliegenden und im Dichtsitz miteinander befindlichen Module zusammengespannt.

Zusätzlich wird eine Spannung dadurch erreicht, daß der Ansatz, der am freien Ende der Hebelarme angeordnet ist und in die schräg gerichtete Rinne des Motorteils eingreift, dieses Motorteil ebenfalls nach unten presst und gegen das darunterliegende Modul im Press-Sitz aufsitzen lässt.

Dadurch wird also eine absolut dichte Dichtverbindung und eine erschütterungsfreie und saubere Dichtpassung zwischen den ineinandergreifenden Modulrändern der übereinander liegenden Module erreicht.

Hierbei wird es im übrigen bevorzugt, wenn im Bereich dieser Dichtflächen noch eine ringsumlaufende Dichtschnur eingebaut ist.

Diese Dichtschnur verhindert die Vibrationsübertragung von dem einen Modul auf das darüber oder darunter liegende andere Modul.

Nach einem weiteren wesentlichen Merkmal der Erfindung ist es vorgesehen, daß alle Module doppelwandig ausgebildet sind, d.h. sie haben eine Innenwand und eine Außenwand. Hierbei wird es bevorzugt, wenn der Zwischenraum zwischen der Innen- und der Außenwand durch ein schallschluckendes Material ausgefüllt ist, wie z.B. einem Kunststoffschaum, Epoxyschaum oder anderes mehr.

Nach einer weiteren wesentlichen Ausgestaltung der Erfindung ist es vorgesehen, daß die einzelnen Filter über leicht lösbare Klemmhalter in den zugeordneten Modulen gehalten werden, wobei die Filterhalter unter Reibungsschluß mit zugeordneten Spannelementen im Zwischenraum zwischen der Außenwand und der Innenwand eingesetzt sind und im Bereich von vertikal gerichteten Längsnuten in der jeweiligen Innenwand des jeweiligen Moduls verschiebbar und feststellbar angeordnet sind.

Damit die Module leicht auswechselbar sind, ist es im übrigen vorgesehen, daß an der Innenfläche der jeweiligen Schwenkhebel Aufnahmeräume für die Führung elektrischer Leitungen vorgesehen sind, so daß diese Leitungen von dem bodennahen Sockelteil in den Innenräumen der Schwenkarme nach oben zu den einzelnen Modulen geführt werden können, wobei die Leitungen dann von der Innenseite des jeweiligen Schwenkhebels von außen her in den modulartig ausgebildeten Ventilatorteil eingeführt werden. Man vermeidet daher im Innenraum entlanggeführte Stromleitungen und Verbindungen, welches ein Auseinanderziehen und Entfernen der einzelnen Module erschwerden könnten.

Im übrigen wird es bevorzugt, wenn der Bedienungsteil bevorzugt im Bereich der horizontal gerichteten Ansätze der Schwenkhebel angeordnet ist, weil damit auch die dem Bedienungsteil zugeordneten Leitungen in dem jeweiligen Schwenkhebel entlanggeführt werden können, ohne daß die Notwendigkeit besteht, die Leitungen für das Bedienteil durch das modulartige Gehäuse hindurchzuführen.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von mehrere Ausführungswege darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: schematisiert ein Luftreinigungsgerät nach der Erfindung in zusammengebautem Zustand,
- Figur 2:: die Ausführung nach Figur 1 in geöffnetem Zustand, mit Schnitt-Darstellung von beidseitig in Ausschwenkposition befindlichen Schwenkhebel,
- Figur 3:: die Ausführung nach Figur 2 bei voneinander abgehobenen Modulen,
- Figur 4:: der Schnitt über zwei modulartig übereinander angeordnete Filterelemente,
- Figur 5:: die Draufsicht auf Figur 4 unter Weglassung des Filterelements,
- Figur 6:: schematische Schnittdarstellung des Luftreinigungsgeräts vor/in Deckenanordnung.
- Figur 7:: schematische Schnittdarstellung eines Punktabsauggeräts mit Schlauchanordnung,
- Figur 8:: schematische Schnittdarstellung eines Ventilations- oder Überdruckgeräts,
- Figur 9:: schematische Schnittdarstellung eines Reinluft-Luftreinigungsgeräts mit speziellem Ausströmteil.

Das Luftreinigungsgerät nach der Erfindung besteht aus einem Gesamtgehäuse 1, welches aus verschiedenen, übereinander angeordneten Modulen besteht. In einem Sockel 2 sind seitliche (nicht näher dargestellte) Ansaugöffnungen vorgesehen, über welche die Luft in den Pfeilrichtungen 3,4 in den Sockel 2 eingesaugt wird. Die Luft strömt dann in Pfeilrichtung 28 nach oben. Direkt auf dem Sockel ist ein erstes Modul 5 im Dichtsitz auf dem Sockel 2 aufgesetzt, wobei dieses Modul 5 einen Filterhalter 6 und ein darin leicht auswechselbar gehaltenes Filter 29 aufweist. Oberhalb dieses Moduls 5 ist ein weiteres Modul 7 angeordnet, welches als Ansaugwanne 8 ausgebildet ist, wobei diese Ansaugwanne 8 über nicht näher dargestellte Befestigungsmittel mit dem darüber lagernden Modul 9 verbunden ist, welches als Ventilatorgehäuse 10 zur Aufnahme eines Ventilators ausgebildet ist.

Oberhalb des Moduls 9 des Ventilatorgehäuses 10 ist ein weiteres Modul 11 angeordnet, welches einen Filterhalter 12 aufweist, in den bevorzugt ein Feinstaubfilter 34 angeordnet ist.

Oberhalb dieses Moduls 11 ist im Dichtsitz ein Ausströmteil 13 angeordnet, welches umlaufende Ausströmgitter 14 aufweist, durch welches die Luft in den Pfeilrichtungen 15 und 16 und ggf. auch nach oben in Pfeilrichtung 17 ausströmt. Die gesamten Teile 2,5,7,9,11,13 sind also turmartig übereinander aufgebaut, greifen im Dichtsitz ineinander und sind leicht lösbar voneinander ausgestaltet, mit Ausnahme, daß bevorzugt die Ansaugwanne 8 mit dem Ventilatorgehäuse 10 dauerhaft verbunden ist.

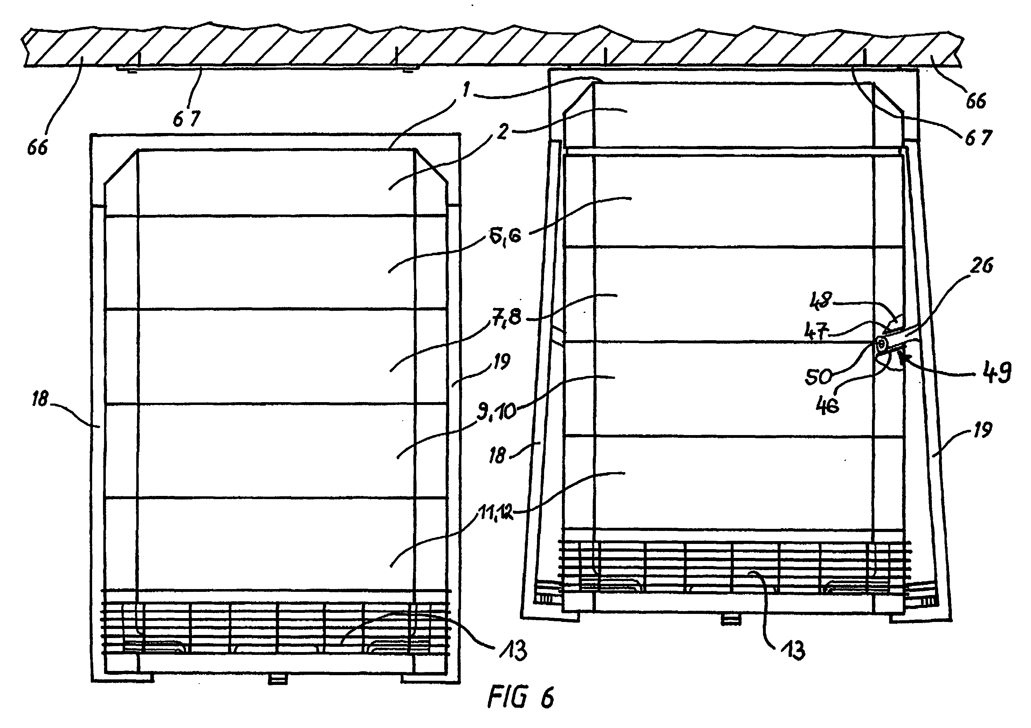

Um die genannten Teile zueinander zu spannen, sind zwei einander entgegengesetzt angeordnete Schwenkhebel 18,19 vorgesehen, wobei jeder Schwenkhebel 18,19 eine untere Schwenkaufnahme 20 aufweist, die in Figur 6 näher dargestellt ist.

Jeder Schwenkhebel 18,19 weist einen etwa horizontal einwärts gerichteten Ansatz 21 auf, an dessen Oberseite bevorzugt das Bedienteil des gesamten Luftgeräts angeordnet ist. Wird der Schwenkhebel 18,19 in den Pfeilrichtungen 23,24 auseinandergezogen, dann kommt der jeweilige Ansatz 21 außer Eingriff mit einer nicht näher dargestellten Ausnehmung 22 im Bereich des Luftausströmteiles 13, und damit können alle Module 5,7,9,11 nach oben in Pfeilrichtung 28 abgehoben werden.

In einer besonderen Weiterbildung der Erfindung ist es aber zusätzlich vorgesehen, daß durch das Verschwenken der Schwenkhebel 18,19 in den Pfeilrichtungen 23,24 noch zusätzlich eine Abhebebewegung der einzelnen Module voneinander in Pfeilrichtung 28 erfolgt. Hierbei wird unter Bezugnahme auf die Figur 6 dargestellt, daß an der jeweiligen Innenseite des jeweiligen Schwenkhebels 18,19 jeweils ein einwärts gerichteter Hebelarm 25,26 angeordnet ist, der an seinem vorderen freien Ende jeweils mit einem Ansatz 50 verbunden ist. Dieser Ansatz 50 greift hierbei in eine Rinne 49 ein, die im Zwischenraum zwischen dem Modul 9 des Ventilatorgehäuses 10 und dem darunter angeordneten Modul 7 der Ansaugseite 8 angeordnet ist.

Die Bodenfläche der Rinne 49 wird durch eine Abdrückfläche 46 gebildet, die schräg einwärts nach oben geneigt ist, wobei diese Abdrückfläche 46 und der untere Teil der Rinne der Ansaugwanne 8 zugeordnet ist, während die Deckfläche der Rinne 49 als Abdrückfläche 47 ausgebildet ist, die in ihrer Neigung etwa parallel der unteren Abdeckfläche 46 angepasst ist.

Die obere Abdrückfläche 47 ist hierbei dem oberen Modul (Ventilatorgehäuse 10) zugeordnet und ist im Bereich einer Ausnehmung 48 im oberen Modulbock 10 ausgebildet.

Wird nun der Schwenkhebel 19 in Pfeilrichtung 23 um seine untere Schwenkaufnahme 20 verschwenkt, dann drückt sich der Ansatz 50 am freien, vorderen Ende des Hebelarmes 26 an der oberen Abdrückfläche 47 ab, und damit wird der gesamte Modulaufbau aus den Modulen 7,9 nach oben in Pfeilrichtung 28 angehoben.

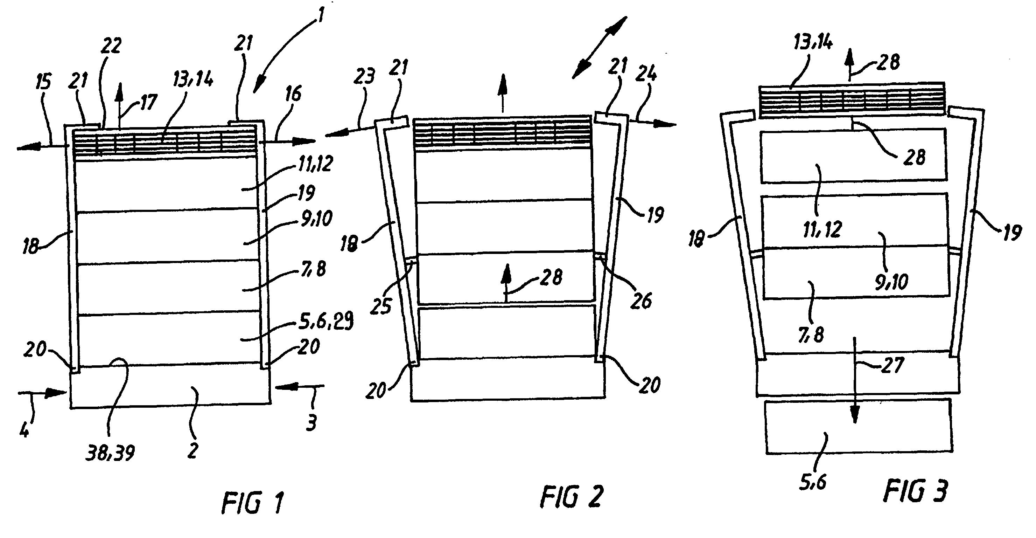

Damit kommt es zu der in Figur 3 dargstellten Abhebebewegung zwischen dem Modulaufbau der Module 7,9 und dem darunter liegenden Modul 5.

Das Modul 5 kann somit leicht aus dem turmartigen Aufbau in Pfeilrichtung 27 in der Art einer Schublade in horizontaler Richtung herausgezogen werden.

Die Figur 3 zeigt zwar die Entfernung in vertikaler Richtung, dies ist aber nur aus zeichnerischen Gründen so dargestellt worden. In Wirklichkeit wird also das Modul 5 in horizontaler Richtung in Pfeilrichtung 27 von dem darunterliegenden Sockel 2 nach außen abgezogen.

Es kann somit leicht aus dem Aufbau herausgelöst werden, weil die darüber liegenden Module 7,9 in Richtung der Abhebebewegung (Pfeilrichtung 28) nach oben abgehoben wurden.

Damit ist eine leichte Auswechselbarkeit des unteren Moduls 5 mit dem darin bevorzugt enthaltenen, vorzugsweise als Grobstaubfilter ausgebildeten Filter 29, gegeben.

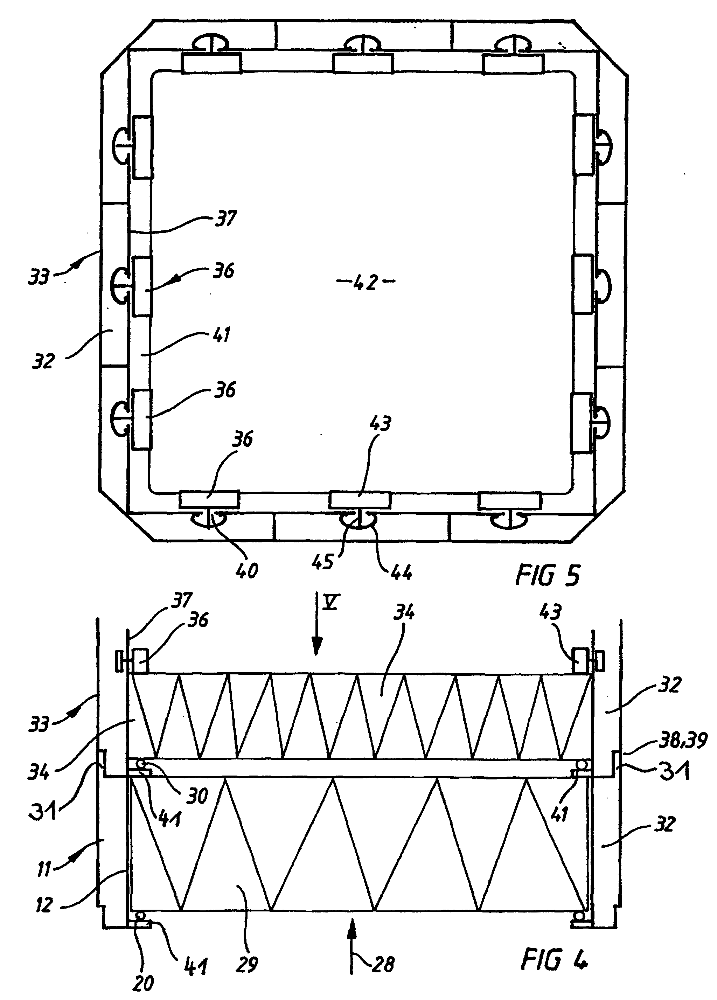

Gleichzeitig kann jetzt aber auch das obere Modul 11 von dem nach oben abgehobenen Modulaufbau der Module 7,9 entfernt werden, wie in Figur 3 gezeigt oder in Erweiterung der Figur 3, auf das oberste Modul 11 noch ein weiteres Modul 33 aufsetzbar ist, welches ebenfalls der Figur 4 entnehmbar einen Filter 34 (Feinfilter) enthält, der in einem entsprechenden Filterhalter 36 gehalten ist.

In Figur 4 zeigt als Erweiterung der Figur 3, daß auf das oberste Modul 11 noch ein weiteres Modul 33 aufsetzbar ist, welches ebenfalls einen Filter 34 enthält, der in einem entsprechenden Filterhalter gehalten ist.

Hierbei würden dann die Schwenkhebel 18,19 gegen gleichartige, länger ausgebildete Schwenkhebel ausgetauscht werden, und auf dem gesamten, nun höher ausgebildeten Aufbau würde dann wiederum das Ausströmteil 13 dichtend aufgesetzt werden und könnte dann - genau wie vorher beschriebenzusammengespannt werden.

Die Figur 4 zeigt den Dichtungssitz der einzelnen Module, was für alle in dem vorher gezeigten Ausführungsbeispiel Figur 1 bis Figur 3 ebenfalls zutrifft und gilt.

Hierbei ist erkennbar, daß jedes Modul aus einem etwa wannenförmigen Aufbau besteht und doppelwandig ausgebildet ist. Es weist jeweils eine Außenwandung auf und eine ringsumlaufende oder teilweise umlaufende Innenwandung, die als Filterhalterung ausgebildet ist.

Die Einzelheiten dieses Aufbaus werden anhand der Figur 5 und anhand des oberen Moduls 33 näher beschrieben.

Es ist also eine ringsumlaufende, insich geschlossene, Außenwand vorhanden, die über einen Zwischenraum 32 von einer inneren, ringsumlaufenden Innenwand 37 beabstandet ist.

Bevorzugt wird dieser Innenraum 32 ausgeschäumt oder mit anderen geräuschdämmenden Medien ausgefüllt.

In der Innenwand 37 sind nun in vertikaler Richtung verlaufende Längsnuten 40 ausgebildet, die parallel zueinander einen gegenseitigen Abstand einnehmen. In diese Längsnuten 40 ist jeweils klemmend ein Filterhalter 36 eingesetzt, der im wesentlichen aus einem inneren Klemmklotz 43 und aus einem mit dem Klemmklotz 43 werkstoffeinstückig verbundenen Steg 45 besteht, der durch die jeweilige Längsnut 40 hindurchgreift und der an seinem äußeren Ende mit einem etwa U-förmigen Spannelement 44 verbunden ist, welches sich mit seinen freien, äußeren Enden klemmend und unter Reibschluß an den Außenflächen der Innenwandung 37 anlegt.

Das Modul 33 bildet eine innere, zentrale Ausnehmung 42, die nach außen hin in Richtung zur Innenwand 37 von einer ringsumlaufenden, etwa horizontal geneigten Anschlagwand 41 begrenzt wird. Auf diese Anschlagwand 41 wird das in Figur 5 nicht näher dargestellte Filter 34 aufgelegt, wobei alle Klemmklötze 43 aus ihren Längsnuten 40 entfernt sind. Nach dem Einlegen des Filters 34 in die Ausnehmung 42, wobei sich das Filter mit seinem Außenrand dichtend an der Anschlagwand 41 anlegt, werden die Klemmklötze 43 in die Längsnuten 40 eingesetzt und nach unten verschoben, so daß sich die Klemmklötze 43 dann unter Klemmwirkung, wie in Figur 4 gezeigt, an dem oberen umlaufenden Rand des Filters 34 anlegen.

Zur besseren Abdichtung des Filters 34 ist es noch vorgesehen, daß zwischen der Unterseite des Filters und der zugeordneten Anschlagwand 41 jeweils ein ringsumlaufender Dichtring 30 angeordnet ist.

Auf diese Weise sind zwei Filter 29,34 turmartig übereinander gebaut und können leicht ausgewechselt werden.

Im übrigen weist jede Modulwanne einen abgekröpften Ansatz 31 auf, der einen Zwischenraum 38 mit einer einliegenden Rundschnur 39 bildet, wobei in diesem Zwischenraum 38 die Außenwand des darunterliegenden Moduls dichtend eingreift, wie in Figur 4 dargestellt.

Ein weiterer Vorteil des Luftreinigungsgeräts - nämlich die Möglichkeit seiner zusätzlichen Decken-Aufhängung - ist in der Figur 6 dargestellt, wobei hier das Luftreinigungsgerät mit dem Gehäuse 1 an einem an der Decke 66 befestigten Montageblech 67 mit seinem Sockel 2 eingerastet wird, so daß das Luftreinigungsgerät kopfüber an der Decke 66 sicher und einfach abnehmbar angehängt ist.

Auch bei dieser Deckenmontage des Luftreinigungsgeräts ist der einfache Filterwechsel aus dem Filtermodul 5 gewährleistet, da dieses auch beim vollständigen Öffnen der beidseitigen Schwenkhebel 18,19 in Pfeilrichtung 23,24 gehalten wird. Auch das zwischen dem Ausströmteil 13 und dem Ventilatorgehäuse 10 angeordnete Filtermodul 11 wird bei nicht vollständigem Öffnen der beidseitigen Schwenkhebel 18,19 in Pfeilrichtung 23,24 durch den Ansatz 21 des jeweiligen Schwenkhebels 18,19 so gehalten, daß der Filter 34 aus dem Filterhalter 12 dieses Moduls 11 problemlos entnehmbar ist.

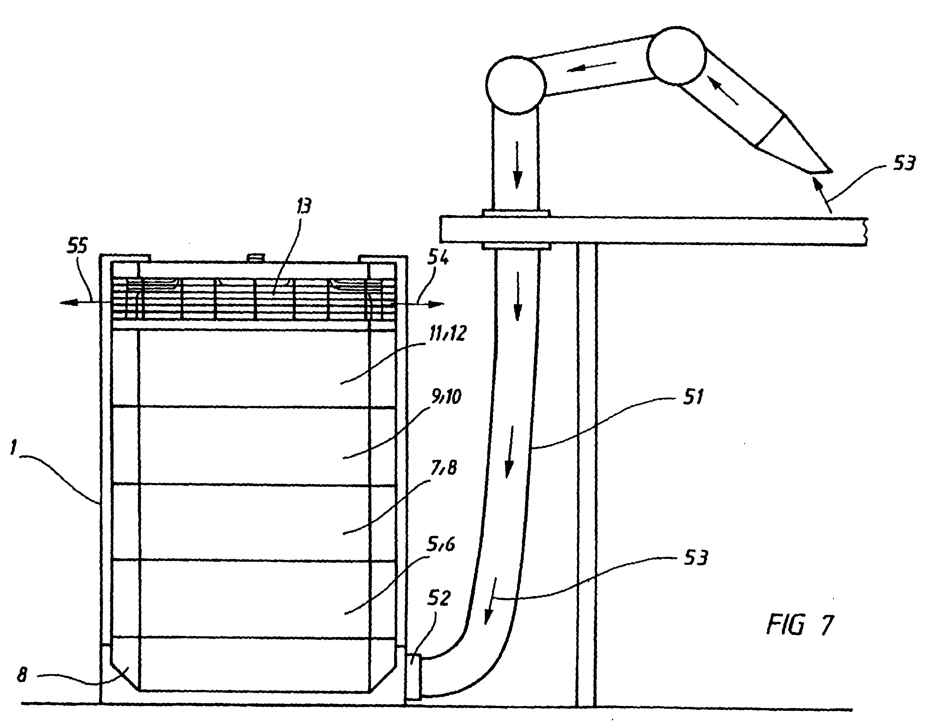

Eine spezielle Ausführungsform des Luftreinigungsgeräts als Punktabsauggerät ist aus der Figur 7 entnehmbar. Hierzu ist das Luftreinigunsgerät mit einem, an einer Seite des Sockels 2 am Gehäuse 1 ausgebildeten Punktabsauganschluss 52 zum Anschluss des Absaugschlauchs 51 versehen, wobei die Luft von dem abzusaugenden Objekt durch den Absaugschlauch 51 in Pfeilrichtung 53 durch den Punktabsauganschluss 52 in das Gehäuse 1 des Luftreinigungsgeräts gelangt und dort durch die einzelnen Module 5,7,9,11 hindurchströmt und das Gehäuse 1 durch das Ausströmteil 13 in der Pfeilrichtung 54, 55 verlässt.

Ein derart ausgebildetes Punktabsauggerät ist vielseitig anwendbar, beispielsweise auch zur Verbesserung des Arbeitsschutzes im Industriebereich, insbesondere auch bei der Schweissrauch-Absaugung, geeignet.

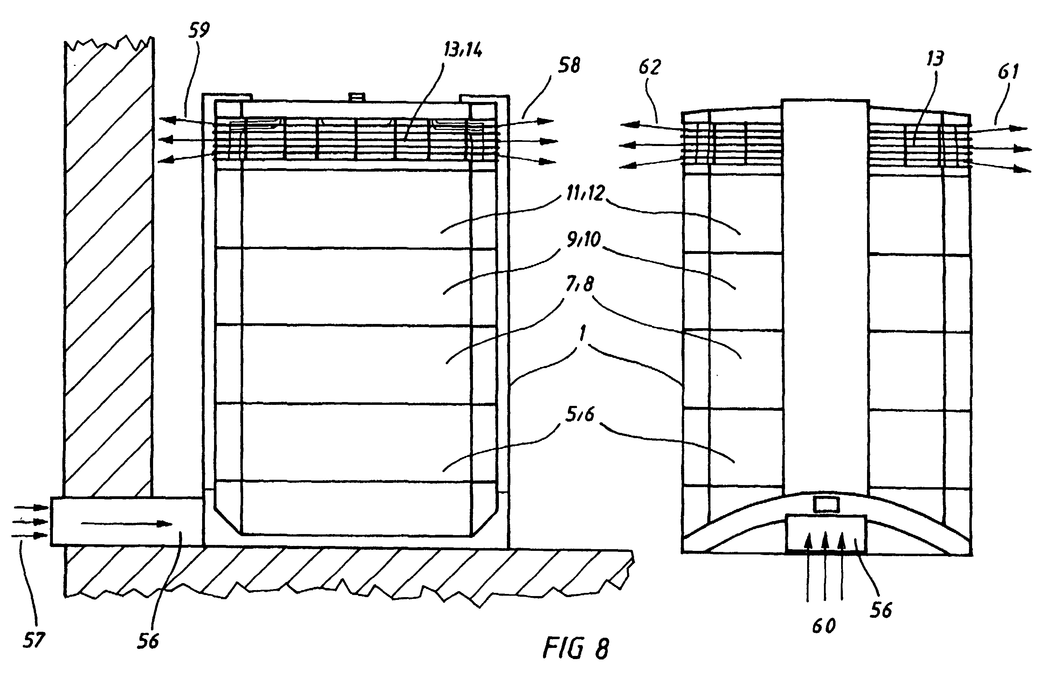

Eine weitere vorteilhafte Einsatzmöglichkeit des erfindungsgemässen Luftreinigungsgeräts als Ventilations- oder Überdruckgerät ist in Figur 8 dargestellt. Hierzu wird ein an einer Seite des Gehäuses 1 im Bereich des Sockels 2 oder unterhalb des bogenförmig gewölbten Gehäuses 1 angeordneter und von der Aussenluft in Pfeilrichtung 57,60 durchströmter Kanal 56 mit dem, hier nicht näher dargestellten Ansaugschluss des Luftreinigungsgeräts verbunden, so daß die in das Gerät in Pfeilrichtung 57,60 durch den Kanal 56 in das Gehäuse 1 des Reinigungsgeräts einströmende, sauerstoffreiche Luft durch die Filtermodule 5,11 gereinigt wird und das Gehäuse 1 durch das Ausströmteil 13 in Pfeilrichtung 58,59,
61, 62 gereinigt verlässt.

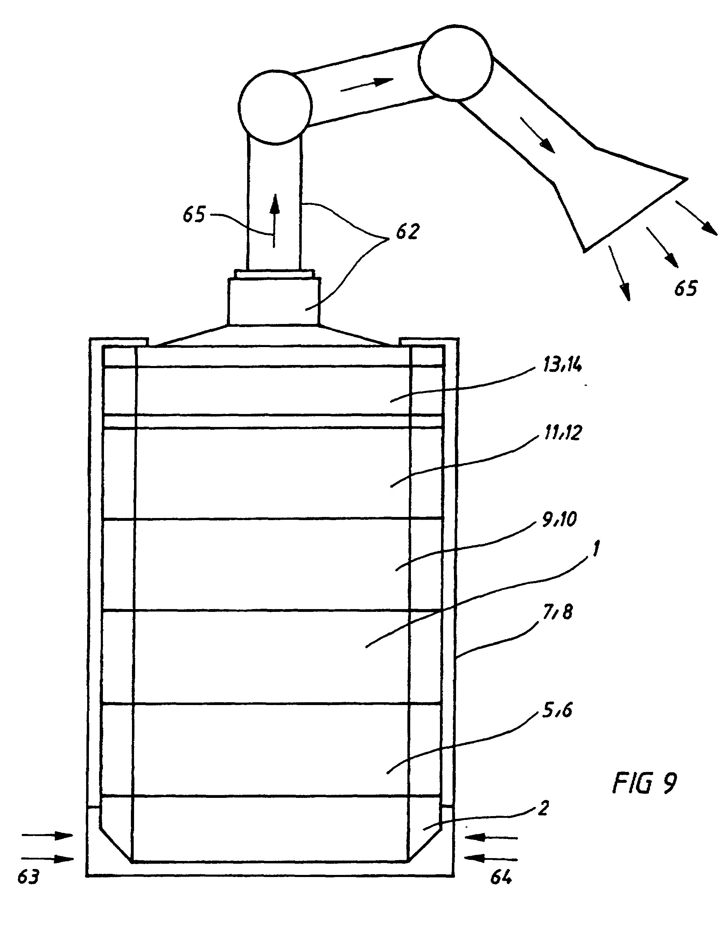

Schließlich ist der des weiteren vorteilhafte Einsatz des Luftreinigungsgeräts als Reinluft-Reinigungsgerät in Figur 9 dargestellt.

Hierzu ist das Ausströmteil 13 des Gehäuses 1 des Luftreinigungsgeräts durch ein spezielles Ausströmteil 62, welches endseitig mit einem Trichter, Schlauch oder Kanal ausgebildet ist, ersetzt. Damit kann die in Pfeilrichtung 63,64 in das Gehäuse 1 des Luftreinigungsgeräts von unten einströmende und durch die Filtermodule 5,11 gereinigte und durch das spezielle Ausströmteil 62 in Pfeilrichtung 65 ausströmende Luft zielgerichtet auf das zu reinigende Objekt gerichtet werden.

Das Gesamtgehäuse 1 ist relativ groß dimensioniert, wodurch sich große Lüftungsquerschnitte ergeben. In einer bevorzugten Ausführungsform hat das Gehäuse 1 Aussenabmessungen von etwa 37 x 37 cm, wodurch sich große Lüftungsquerschnitte und ein entsprechendes leises Lüftungsgeräusch ergibt.

Das Gesamtgehäuse 1 ist relativ groß dimensioniert, wodurch sich große Lüftungsquerschnitte ergeben. In einer bevorzugten Ausführungsform hat das Gehäuse Außenabmessungen von etwa 37 x 37 cm, wodurch sich große Lüftungsquerschnitte und entsprechend ein leises Lüftungsgeräusch ergibt.

### Zeichnungs-Legende

- 1: Gesamtgehäuse
- 2: Sockel
- 3: Pfeilrichtung
- 4: "
- 5: Modul
- 6: Filterhaler
- 7: Modul
- 8: Ansaugwanne
- 9: Modul
- 10: Ventilatorgehäuse
- 11: Modul
- 12: Filterhalter
- 13: Ausströmteil
- 14: Ausströmgitter
- 15: Pfeilrichtung
- 16: "
- 17: "
- 18: Schwenkhebel
- 19: "
- 20: Schwenkaufnahme
- 21: Ansatz
- 22: Ausnehmung
- 23: Pfeilrichtung

- 24: "
- 25: Hebelarm

- 26: Hebelarm
- 27: Pfeilrichtung
- 28: Pfeilrichtung
- 29: Filter
- 30: Dichtring
- 31: Ansatz
- 32: Zwischenraum
- 33: Modul
- 34: Filter (Feinfilter)

- 36: Filterhalter
- 37: Innenwand
- 38: Zwischenraum
- 39: Rundschnur
- 40: Längsnut
- 41: Anschlagwand
- 42: Ausnehmung
- 43: Klemmklotz
- 44: Spannelement
- 45: Steg
- 46: Abdrückfläche (unten)
- 47: Abdrückfläche (oben)
- 48: Ausnehmung (oberer Modulblock)
- 49: Rinne (unterer Modulblock)
- 50: Ansatz
- 51: Absaugschlauch
- 52: Punktabsauganschluß
- 53: Pfeilrichtung
- 54: Pfeilrichtung
- 55: "
- 56: Kanal
- 57: Pfeilrichtung

- 58: "
- 59: "
- 60: "
- 61: "
- 62: Ausströmteil
- 63: Pfeilrichtung
- 64: "
- 65: Pfeilrichtung
- 66: Decke
- 67: Montageblech

## Patentansprüche

1. Luftreinigungsgerät im wesentlichen bestehend aus einem Ansaugteil, einem Ventilatorgehäuse mit einem oder mehreren Ventilatoren und aus einem oder mehreren, vor oder nach dem Ventilator angeordneten, Luftfilter, wobei das Gehäuse(1) einen Sockel (2) aufweist und turmartig aus übereinander angeordneten, im Dichtsitz ineinandergreifenden und voneinander leicht lösbaren, modulartig ausgebildeten Funktionsteilen (5,7,9,11,13) ausgebildet ist, **dadurch gekennzeichnet, daß** das Gehäuse (1) mindestens einen, an seinem seitlichen Teil mittels einer Schwenkaufnahme (20) in Richtung vom Gehäuse (1) wegschwenkbar angeordneten, alle Teile (2,5,7,9,11,13) des Gehäuses (1) von außen her übergreifenden und diese zusammenspannenden, Schwenkhebel (18,19) aufweist.

2. Luftreinigungsgerät nach Anspruch 1, **dadurch gekennzeichnet, daß** vorzugsweise zwei, einen gegenseitigen Abstand zueinander einnehmenden und mittels Schwenkaufnahme (20) am seitlichen Teil des Gehäuses (1) zueinander schwenkbar und in Richtung seitlich vom Gehäuse (1) weg ausschwenkbar gelagerten, den turmartigen Aufbau der einzelnen Teile (2,5,7,9,11,13) des Gehäuses (1) von außen her übergreifenden und diese zusammenspannenden, Schwenkhebel (18,19) Verwendung finden.

3. Luftreinigungsgerät nach Anspruch 1, **dadurch gekennzeichnet, daß** jedes Modul (5,7,9,11,13) in der Art einer in horizontaler Richtung herausziehbaren und damit schnell, auch untereinander auswechselbaren Schublade ausgebildet ist.

4. Luftreinigungsgerät nach Anspruch 1, **dadurch gekennzeichnet, daß** alle in den Modulen (5,11,33) im jeweiligen Filterhalter (6,12,36) angeordneten Filter (29,34) sowie der Motorteil oder alle anderen Funktionsteile schnell auswechselbar angeordnet sind.

5. Luftreinigungsgerät nach Anspruch 1 oder 2, **dadurch gekennzeichnet, daß** die Schwenkhebel (18,19) mit unterschiedlicher Länge, wodurch die Aufbauhöhe des modulartigen Gesamtgehäuses (1) beliebig veränderbar ist, Verwendung finden.

6. Luftreinigungsgerät nach Anspruch 1, **dadurch gekennzeichnet, daß** der Sockel (2) seitliche Ansaugöffnungen aufweist, über welche Luft in Richtung seitlich zum Gehäuse (1) hin in den Sockel (2) eingesaugt wird, welche dann durch das Luftreinigungsgerät hindurch nach oben strömt.

7. Luftreinigungsgerät nach Anspruch 1, **dadurch gekennzeichnet, daß** das erste Modul (5) direkt im Dichtsitz auf dem Sockel (2) aufgesetzt ist und einen Filterhalter (6) mit leicht auswechselbarem, vorzugsweise als Grobstaubfilter ausgebildeten Filter (29) aufweist.

8. Luftreinigungsgerät nach Anspruch 1, **dadurch gekennzeichnet, daß** auf dem ersten Modul (5) ein weiteres Modul (7) aufgesetzt ist, welches als Ansaugwanne (8) ausgebildet und mittels Befestigungsmittel mit dem darüber lagernden Modul (9) mit Ventilatorgehäuse (10) vorzugsweise durch Verschrauben dauerhaft fest angeordnet ist.

9. Luftreinigungsgerät nach Anspruch 1, **dadurch gekennzeichnet, daß** oberhalb des als Ventilatorgehäuse (10) ausgebildeten Moduls (9) das weitere Modul (11) mit in dem Filterhalter (12) befindlichen Feinstaubfilter (34), angeordnet ist.

10. Luftreinigungsgerät nach Anspruch 9, **dadurch gekennzeichnet, daß** oberhalb dieses Moduls (11) im Dichtsitz das Ausströmteil (13) mit umlaufendem Ausströmgitter (14) angeordnet ist, durch welches die Luft in seitliche Richtungen vom Gehäuse (1) weg, und ggf. auch nach oben hin vom Gehäuse (1) weg ausströmt.

11. Luftreinigungsgerät nach Anspruch 1, **dadurch gekennzeichnet, daß** auf das Modul (11) noch ein weiteres Modul (33) mit einem Filter (34) und auf diesem dann das Ausströmteil (13) mit umlaufendem Ausströmgitter (14) aufsetzbar ist, und jedes Modul (5,7,9,11,33) aus einem etwa wannenförmigen Aufbau besteht und mit einer in sich geschlossenen Aussenwand, die über einen, vorzugsweise ausgeschäumten, Zwischenraum (32) von einer inneren, ringsum laufenden Innenwand (37) beabstandet ist, versehen ist.

12. Luftreinigungsgerät nach Anspruch 1 und 11, **dadurch gekennzeichnet, daß** in der Innenwand (37) in vertikaler Richtung verlaufende, parallel zueinander einen gegenseitigen Abstand einnehmende, jeweils klemmend die Filterhalter (6,12,36) tragende, Längsnuten (40) ausgebildet sind.

13. Luftreinigungsgerät nach Anspruch 1 und 11 bis 12, **dadurch gekennzeichnet, daß** der, im wesentlichen aus einem inneren Klemmklotz (43) und aus einem mit dem Klemmklotz (43) werkstoffeinstückig verbundenen Steg (45) bestehende, jeweilige Filterhalter (6,12,36) durch die jeweilige Längsnut (40) hindurchgreift und an seinem äußeren Ende mit einem etwa U-förmigen Spannelement (44) verbunden ist, welches sich mit seinen freien, äußeren Enden klemmend und unter Reibschluß an den Außenflächen der Innenwandung (37) anlegt.

14. Luftreinigungsgerät nach Anspruch 1 und 11-13, **dadurch gekennzeichnet, daß** jedes Filtermodul (5,11,33) eine innere, zentrale Ausnehmung (42) aufweist, die nach außen hin in Richtung zur Innenwand (37) von einer ringsumlaufenden, etwa horizontal geneigten Anschlagwand (41) begrenzt ist, wobei auf diese Anschlagwand (41) nach Entfernen aller Klemmklötze (43) aus ihren Längsnuten (40) das Filter (29,34) auflegbar ist und durch die wieder in ihre jeweilige Längsnut (40) eingesetzten und nach unten verschobenen Klemmklötze (43) verklemmbar ist.

15. Luftreinigungsgerät nach Anspruch 1 und 11-14, **dadurch gekennzeichnet, daß** zur besseren Abdichtung des jeweiligen Filters (29,34), zwischen seiner Unterseite und der zugeordneten Anschlagwand (41) jeweils ein ringsumlaufender Dichtring (30) angeordnet ist.

16. Luftreinigungsgerät nach Anspruch 1, **dadurch gekennzeichnet, daß** jede Modulwanne einen abgekröpften Ansatz (31) aufweist, der einen Zwischenraum (38) mit einer einliegenden Rundschnur (39) bildet, wobei in diesem Zwischenraum (38) die Außenwand des darunterliegenden Moduls dichtend eingreift.

17. Luftreinigungsgerät nach Anspruch 1, **dadurch gekennzeichnet, daß** jeder Schwenkhebel (18,19) eine untere Schwenkaufnahme (20) und einen etwa horizontal einwärts gerichteten, in die Ausnehmung (22) im Bereich des Ausströmteils (13) eingreifenden, Ansatz (21) aufweist, wobei dieser beim Auseinanderziehen der Schwenkhebel (18,19) in die Richtungen seitlich vom Gehäuse (1) weg mit der Ausnehmung (22) außer Eingriff kommt und damit das Abheben aller Module (5,7,9,11) gewährleistet ist.

18. Luftreinigungsgerät nach Anspruch 1 und 17, **dadurch gekennzeichnet, daß** zum Abheben der einzelnen Module (5,7,9,11,33) voneinander, in Richtung axial vom Sockel (2) weg, an den in die Richtungen seitlich vom Gehäuse (1) weg verschwenkten Schwenkhebeln (18,19), an der jeweiligen Innenseite des jeweiligen Schwenkhebels (18,19) jeweils ein einwärts gerichteter Hebelarm (25,26) angeordnet ist, der an seinem vorderen freien Ende jeweils mit einem Ansatz (50) verbunden ist, wobei dieser hierbei in eine Rinne (49) eingreift, die im Zwischenraum zwischen dem Modul (9) des Ventilatorgehäuses (10) und dem darunter angeordneten Modul (7) der Ansaugseite (8) angeordnet ist.

19. Luftreinigungsgerät nach Anspruch 18, **dadurch gekennzeichnet, daß** die Bodenfläche der Rinne (49) durch eine Abdrückfläche (46), die schräg einwärts nach oben geneigt ist, gebildet wird, wobei diese Abdrückfläche (46) und der untere Teil der Rinne der Ansaugwanne (8) zugeordnet ist, während die Deckfläche der Rinne (49) als Abdrückfläche (47) ausgebildet ist, die in ihrer Neigung etwa parallel der unteren Abdrückfläche (46) angepasst ist.

20. Luftreinigungsgerät nach Anspruch 17-19, **dadurch gekennzeichnet, daß** die obere Abdrückfläche (47) dem oberen Modul (Ventilatorgehäuse 10) zugeordnet und hierbei im Bereich einer Ausnehmung (48) im oberen Modulblock (10) ausgebildet ist.

21. Luftreinigungsgerät nach Anspruch 1, **dadurch gekennzeichnet, daß** zur Aufhängung des Luftreinigungsgeräts an einem an der Decke (66) befestigten Montageblech (67) der Sockel (2) des Luftreinigungsgeräts mit zweckentsprechenden Befestigungsmitteln ausgebildet ist, und das derart deckenseitigangeordnete Luftreinigungsgerät selbst bei in Richtung seitlich vom Gehäuse (1) weg bis mindestens zur Hälfte der Schwenkstrecke ausgeschwenkten Schwenkhebeln (18,19) durch den Ansatz (21) des jeweiligen Schwenkhebels (18,19) vor dem Herabfallen gesichert ist.

22. Luftreinigungsgerät nach Anspruch 1, **dadurch gekennzeichnet, daß** die elektrischen Leitungen in den Schwenkarmen (18,19) in - an der Innenfläche des jeweiligen Schwenkhebels zweckentsprechend ausgebildeten - Aufnahmeräumen geführt sind und bei geöffneten Schwenkarmen (18,19) der Motor automatisch abschaltet.

23. Luftreinigungsgerät nach einem der Ansprüche 1 bis 22, **dadurch gekennzeichnet, daß** das Luftreinigungsgerät insbesondere als Punktabsaug-, als Ventilations- oder Überdruck- oder als Reinluft-Reinigungsgerät einsetzbar ist.

24. Luftreinigungsgerät nach Anspruch 1 oder 23, **dadurch gekennzeichnet, daß** das als Punktabsauggerät einestzbare Luftreinigungsgerät mit einem, an einer Seite des Sockels (2) des Gehäuses (1) zum Anschluß eines Absaugschlauches (53) angeordneten Punktabsauganschluß (52) ausgebildet ist.

25. Luftreinigungsgerät nach Anspruch 1 oder 23, **dadurch gekennzeichnet, daß** das als Ventilations- oder Überdruckgerät einsetzbare Luftreinigungsgerät mit einem, an dem Geräte-Ansauganschluß angeschlossenen und von der Luft in seitlicher Richtung und/oder von unten her in den Sockel hinein durchströmten Kanal (56) ausgebildet ist.

26. Luftreinigungsgerät nach Anspruch 1 oder 23, **dadurch gekennzeichnet, daß** das als Reinluft-Reinigungsgerät einsetzbare Luftreinigungsgerät mit einem als Trichter, Schlauch oder Kanal oder dergleichen, ausgebildeten, von der Luft in Richtung nach außen hin durchströmten Ausströmteil (62) ausgebildet ist.

27. Luftreinigungsgerät nach Anspruch 1 oder 23, **dadurch gekennzeichnet, daß** durch das Verschwenken der Schwenkhebel (18,19), in Richtung seitlich vom Gehäuse weg, noch zusätzlich eine Abhebebewegung der einzelnen Module voneinander in Richtung vom Sockel (2)weg erfolgt.

## Claims

1. Air purification apparatus substantially comprising an intake part, a fan housing containing one or more fans and one or more air filters arranged ahead of or behind the fan, wherein the housing (1) possesses a base (2) and is constructed in the manner of a tower from modularly constructed functional parts (5, 7, 9, 11, 13) arranged on top of one another and engaging in one another in the sealing seat and readily detachable from one another, **characterised in that** the housing (1) has at least one pivot lever (18, 19) arranged to be pivoted in the direction away from the housing (1) on a lateral part by means of a pivot receptacle (20), engaging from the outside over all parts (2, 5, 7, 9, 11, 13) of the housing (1) and clamping the latter together.

2. Air purification apparatus according to Claim 1, **characterised in that** preferably two pivot levers (18, 19) assuming a mutual distance relative to one another, pivotable relative to one another by means of a pivot receptacle (20) on the lateral part of the housing (1) and mounted pivotably away from the sides of the housing (1) are used to engage over the tower-like structure of the individual parts (2, 5, 7, 9, 11, 13) of the housing (1) from the outside and to clamp the latter together.

3. Air purification apparatus according to Claim 1, **characterised in that** each module (5, 7, 9, 11, 13) is constructed in the manner of a drawer which can be pulled out in the horizontal direction and hence can be quickly interchanged with another.

4. Air purification apparatus according to Claim 1, **characterised in that** all filters (29, 34) each arranged in a filter holder (6, 12, 36) in the modules (5, 11, 33) and the motor part or all other functional parts are arranged in rapidly interchangeable manner.

5. Air purification apparatus according to Claim 1 or 2, **characterised in that** the pivot levers (18, 19) are used in different lengths by which means the structural height of the entire modular housing (1) is arbitrarily variable.

6. Air purification apparatus according to Claim 1, **characterised in that** the base (2) has lateral intake openings through which air is sucked in at the side of the housing (1) into the base (2) which then flows upwards through the air purification apparatus.

7. Air purification apparatus according to Claim 1, **characterised in that** the first module (5) is set up directly in the sealing seat on the base (2) and possesses a filter holder (6) containing a readily interchangeable filter (29) preferably constructed as a grit filter.

8. Air purification apparatus according to Claim 1, **characterised in that** set up on the first module (5) is another module (7) which is constructed as an intake trough (8) and is arranged durably fixed by means of fastening means to the module (9) with the fan housing (10) mounted thereover preferably by means of screwing.

9. Air purification apparatus according to Claim 1, **characterised in that** above the module (9) constructed as fan housing (10) the further module (11) containing the fine dust filter (34) located in the filter holder (12) is arranged.

10. Air purification apparatus according to Claim 9, **characterised in that** arranged above this module (11) in the sealing seat is the outflow part (13) with all-round outflow grille (14) through which the air flows out in lateral directions away from the housing (1) and possibly also upwards away from the housing (1).

11. Air purification apparatus according to Claim 1, **characterised in that** on the module (11) yet another module (33) having a filter (34) and then on the latter the outflow part (13) with all-round outflow grille (14) is attachable and each module (5, 7, 9, 11, 33) consists of an approximately trough-shaped structure and is provided with an outer wall closed on itself which is held at a distance from an inner all-round inside wall (37) by a preferably foamed-out intermediate space (32).

12. Air purification apparatus according to Claim 1 and 11, **characterised in that** in the inner wall (37) longitudinal grooves (40) running in the vertical direction, assuming a mutual spacing relative to one another and carrying in each case the filter holders (6, 12, 36) are constructed.

13. Air purification apparatus according to Claim 1 and 11 to 12, **characterised in that** each filter holder (6, 12, 36) consisting substantially of an inner clamping block (43) and of a web (45) connected in one piece of material to the clamping block (43) engages through the corresponding longitudinal groove (40) and is connected at its outer end to an approximately U-shaped clamping member (44) which by its free, outer ends fits in clamping manner and with frictional engagement against the outer surfaces of the inner wall (37).

14. Air purification apparatus according to Claim 1 and 11-13, **characterised in that** each filter module (5, 11, 33) has an internal, central recess (42) which is bounded towards the outside in the direction of the inner wall (37) by a limit stop wall (41) running all round and inclined approximately horizontally, wherein on this limit stop wall (41) after removing all clamping blocks (43) from their longitudinal grooves (40) the filter (29, 34) can be laid and can be clamped by the clamping blocks inserted once again into their corresponding longitudinal groove (40) and pushed down.

15. Air purification apparatus according to Claim 1 and 11-14, **characterised in that** for improved sealing off of each filter (29, 34) a sealing ring (30) running all round is arranged in each case between its underside and the associated limit stop wall (41).

16. Air purification apparatus according to Claim 1, **characterised in that** each module trough possesses an offset shoulder (31) which forms an intermediate space (38) having an inlaid round cord (39), wherein the outer wall of the module located thereunder engages in sealing manner in this intermediate space (38).

17. Air purification apparatus according to Claim 1, **characterised in that** each pivot lever (18, 19) has a lower pivot receptacle (20) and a shoulder (21) directed approximately horizontally inwards and engaging in the recess (22) in the region of the outflow part (13), wherein on pulling apart the pivot lever (18, 19) comes out of engagement with the recess (22) in the directions away from the sides of the housing (1) and in this way the lifting off of all modules (5, 7, 9, 11) is ensured.

18. Air purification apparatus according to Claim 1 and 17, **characterised in that** to lift off the individual modules (5, 7, 9, 11, 33) from one another in the direction axially away from the base (2), on the pivot levers (18, 19) pivoted in the directions away from the sides of the housing (1) on each inner side of each pivot lever (18, 19) an inwardly directed lever arm (25, 26) is arranged each of which is connected at its free front end to a shoulder (50), wherein in doing so the latter engages in a channel (49) which is arranged in the cavity between the module (9) of the fan housing (10) and the module (7) of the intake side (8) arranged thereunder.

19. Air purification apparatus according to Claim 18, **characterised in that** the bottom surface of the channel (49) is formed by a press-off surface (46) which is inclined at an upwardly inward slope, wherein this press-off surface (46) and the lower part of the channel is assigned to the intake trough (8) while the cover surface of the channel (49) is constructed as a press-off surface (47) whose inclination is adapted to be approximately parallel to the lower press-off surface (46).

20. Air purification apparatus according to Claim 17-19, **characterised in that** the upper press-off surface (47) is assigned to the upper module (fan housing 10) and at the same time is constructed in the region of a recess (48) in the upper module block (10).

21. Air purification apparatus according to Claim 1, **characterised in that** for suspending the air purification apparatus on a mounting plate (67) fastened to the ceiling (66) the base (2) of the air purification apparatus is constructed with fastening means suitable for the purpose and the air purification apparatus arranged on the ceiling in this way is secured against falling down by the shoulder (21) of the respective pivot lever (18, 19) even when the pivot levers are swivelled out in the direction away from the sides of the housing (1) up to at least half the pivot distance.

22. Air purification apparatus according to Claim 1, **characterised in that** the electric leads are conveyed in accommodating chambers constructed in a manner suitable for the purpose in the pivot arms on the inner surface of the respective pivot lever and when the pivot arms (18, 19) are open the motor switches off automatically.

23. Air purification apparatus according to Claims 1 to 22, **characterised in that** the air purification apparatus can be employed in particular as a point-extraction, ventilation or excess-pressure, or clean air purification apparatus.

24. Air purification apparatus according to Claim 1 or 23, **characterised in that** the air purification apparatus employable as a point-extraction apparatus is constructed with a point extraction connection (52) arranged on a side of the base (2) of the housing (1) for connecting an extraction hose (53).

25. Air purification apparatus according to Claim 1 or 23, **characterised in that** the air purification apparatus employable as a ventilation or excess-pressure apparatus is constructed with a channel (56) connected to the apparatus intake connection through which the air flows in the side direction and/or from below into the base.

26. Air purification apparatus according to Claim 1 or 23, **characterised in that** the air purification apparatus employable as a clean air purification apparatus is constructed with an outflow part (62) constructed in the form of a funnel, hose or channel or the like through which the air flows towards the outside.

27. Air purification apparatus according to Claim 1 or 23, **characterised in that** by swivelling the pivot levers (18, 19) away from the sides of the housing a lift-off movement of the individual modules from one another in the direction away from the base (2) additionally takes place.

## Revendications

1. Purificateur d'air composé essentiellement d'un élément d'aspiration, d'une enveloppe de ventilateur avec un ou plusieurs ventilateurs, et d'un ou plusieurs filtres à air disposés avant ou après le ventilateur, l'enveloppe (1) comportant un socle (2) et étant formée à la manière d'une tour d'éléments fonctionnels modulaires (5, 7, 9, 11, 13) qui sont superposés, qui s'emboîtent de manière étanche et qui peuvent facilement être détachés les uns des autres, **caractérisé en ce que** l'enveloppe (1) comporte au moins un levier pivotant (18, 19) qui est disposé pour pouvoir être éloigné par pivotement de l'enveloppe (1), au niveau de la partie latérale de celle-ci, grâce à un logement de pivotement (20), qui couvre de l'extérieur tous les éléments (2, 5, 7, 9, 11, 13) de l'enveloppe (1) et qui les réunit par serrage.

2. Purificateur d'air selon la revendication 1, **caractérisé en ce qu'**on utilise de préférence deux leviers pivotants (18, 19) qui définissent un écartement mutuel, qui sont aptes à pivoter l'un par rapport à l'autre, au niveau de la partie latérale de l'enveloppe (1), grâce au logement de pivotement (20), qui sont montés pour pouvoir s'éloigner par pivotement, latéralement, de l'enveloppe (1), et qui couvrent de l'extérieur et réunissent par serrage la structure en forme de tour des éléments individuels (2, 5, 7, 9, 11, 13) de l'enveloppe (1).

3. Purificateur d'air selon la revendication 1, **caractérisé en ce que** les modules (5, 7, 9, 11, 13) sont conçus à la manière de tiroirs aptes à être sortis horizontalement et donc à être échangés rapidement, même les uns au-dessous des autres.

4. Purificateur d'air selon la revendication 1, **caractérisé en ce que** tous les filtres (29, 34) disposés dans les modules (5, 11, 33), dans les porte-filtre respectifs (6, 12, 36), ainsi que l'élément pour moteur ou tous les autres éléments fonctionnels sont disposés de manière à pouvoir être échangés rapidement.

5. Purificateur d'air selon la revendication 1 ou 2, **caractérisé en ce qu'**on utilise des leviers pivotants (18, 19) de longueurs différentes, moyennant quoi la hauteur de construction de l'enveloppe totale modulaire (1) peut être modifiée comme on veut.

6. Purificateur d'air selon la revendication 1, **caractérisé en ce que** le socle (2) présente des ouvertures d'aspiration latérales par lesquelles l'air est aspiré dans le socle (2), latéralement en direction de l'enveloppe (1), et s'écoule ensuite vers le haut à travers le purificateur d'air.

7. Purificateur d'air selon la revendication 1, **caractérisé en ce que** le premier module (5) est posé directement sur le socle (2), de manière étanche, et comporte un porte-filtre (6) avec un filtre (29) facile à échanger et conçu de préférence comme un filtre pour les grosses poussières.

8. Purificateur d'air selon la revendication 1, **caractérisé en ce qu'**il est prévu, posé sur le premier module (5), un autre module (7) qui est conçu comme une cuvette d'aspiration (8) et qui est relié à demeure à l'aide de moyens de fixation, de préférence par vissage, au module (9) placé sur lui et comportant une enveloppe de ventilateur (10).

9. Purificateur d'air selon la revendication 1, **caractérisé en ce qu'**il est prévu, au-dessus du module (9) conçu comme une enveloppe de ventilateur (10), le module suivant (11) avec un filtre pour fines poussières (34) qui se trouve dans le porte-filtre (12).

10. Purificateur d'air selon la revendication 9, **caractérisé en ce qu'**on dispose de manière étanche au-dessus de ce module (11) l'élément d'évacuation (13) avec une grille d'évacuation périphérique (14), par lequel l'air sort de l'enveloppe (1) dans des directions latérales et éventuellement aussi vers le haut.

11. Purificateur d'air selon la revendication 1, **caractérisé en ce qu'**on peut poser sur le module (11) encore un autre module (33) avec un filtre (34) puis, sur celui-ci, l'élément d'évacuation (13) avec la grille d'évacuation périphérique (14), et chaque module (5, 7, 9, 11, 33) se compose d'une structure à peu près en forme de cuvette et est pourvu d'une paroi extérieure fermée qui est espacée grâce à un espace intermédiaire (32), de préférence garni de mousse, d'une paroi intérieure (37) prévue sur tout le tour.

12. Purificateur d'air selon les revendications 1 et 11, **caractérisé en ce qu'**on forme dans la paroi intérieure (37) des rainures longitudinales (40) qui s'étendent dans le sens vertical, qui définissent, parallèlement les unes aux autres, un espacement mutuel et qui portent par serrage les porte-filtre respectifs (6, 12, 36).

13. Purificateur d'air selon les revendications 1 et 11 à 12, **caractérisé en ce que** le porte-filtre (6, 12, 36), composé essentiellement d'un bloc de serrage intérieur (43) et d'une nervure (45) reliée d'une seule pièce à celui-ci, traverse la rainure longitudinale respective (40) et est relié, à son extrémité extérieure, à un élément de serrage à peu près en U (44) qui s'applique par serrage et par friction, avec ses extrémités extérieures libres, contre les surfaces extérieures de la paroi intérieure (37).

14. Purificateur d'air selon les revendications 1 et 11 à 13, **caractérisé en ce que** chaque module de filtre (5, 11, 33) comporte un creux central intérieur (42) qui est délimité vers l'extérieur, en direction de la paroi intérieure (37), par une paroi de butée périphérique (41), inclinée à peu près à l'horizontale, étant précisé que le filtre (29, 34) peut être posé sur cette paroi de butée (41) après qu'on a enlevé tous les blocs de serrage (43) de leurs rainures longitudinales (40), et est apte à être serré grâce aux blocs de serrage (43) replacés dans leur rainure longitudinale respective (40) et déplacés vers le bas.

15. Purificateur d'air selon les revendications 1 et 11 à 14, **caractérisé en ce que** pour une meilleure étanchéité de chaque filtre (29, 34), on dispose entre son côté inférieur et la paroi de butée associée (41) une bague d'étanchéité périphérique (30).

16. Purificateur d'air selon la revendication 1, **caractérisé en ce que** chaque cuvette modulaire comporte une saillie coudée (31) qui définit un espace intermédiaire (38) avec un cordon torique intégré (39), la paroi extérieure du module situé au-dessous s'emboîtant de manière étanche dans cet espace intermédiaire (38).

17. Purificateur d'air selon la revendication 1, **caractérisé en ce que** chaque levier pivotant (18, 19) comporte un logement de pivotement inférieur (20) et une saillie (21) dirigée vers l'intérieur à peu près horizontalement et pénétrant dans le creux (22) prévu dans la zone de l'élément d'évacuation (13), laquelle saillie (21), lorsque les leviers pivotants (18, 19) sont écartés et éloignés latéralement de l'enveloppe (1), cesse d'être en contact avec le creux (22) et garantit ainsi le soulèvement de tous les modules (5, 7, 9, 11).

18. Purificateur d'air selon les revendications 1 et 17, **caractérisé en ce que** pour soulever les modules individuels (5, 7, 9, 11, 33) les uns par rapport aux autres, on prévoit, en s'éloignant axialement du socle (2), sur les leviers pivotants (18, 19) qui ont pivoté pour s'éloigner latéralement de l'enveloppe (1) et sur le côté intérieur de chaque levier pivotant (18, 19), un bras de levier (25, 26) dirigé vers l'intérieur qui est relié, à son extrémité libre avant, à une saillie (50), laquelle saillie (50) pénètre dans une rainure (49) qui est disposée dans l'espace intermédiaire entre le module (9) de l'enveloppe de ventilateur (10) et le module (7), disposé au-dessous, du côté aspiration (8).

19. Purificateur d'air selon la revendication 18, **caractérisé en ce que** la surface inférieure de la rainure (49) est définie par une surface d'éjection (46) qui est inclinée vers l'intérieur et vers le haut, cette surface d'éjection (46) et la partie inférieure de la rainure étant associées à la cuvette d'aspiration (8) tandis que la zone supérieure de la rainure (49) est conçue somme une surface d'éjection (47) dont l'inclinaison est adaptée à peu près parallèlement à la surface d'éjection inférieure (46).

20. Purificateur d'air selon les revendications 17 à 19, **caractérisé en ce que** la surface d'éjection supérieure (47) est associée au module supérieur (enveloppe de ventilateur 10) et est formée dans la zone d'un creux (48), dans le bloc modulaire supérieur (10).

21. Purificateur d'air selon la revendication 1, **caractérisé en ce que** pour accrocher ledit purificateur d'air à une tôle de montage (67) fixée au plafond (66), on prévoit sur son socle (2) des moyens de fixation adéquats, et le purificateur d'air ainsi disposé côté plafond, même quand les leviers pivotants (18, 19) ont été éloignés par pivotement, latéralement, de l'enveloppe (1) jusqu'à la moitié au moins de la course de pivotement, est protégé à l'encontre d'une chute grâce à la saillie (21) des leviers pivotants (18, 19).

22. Purificateur d'air selon la revendication 1, **caractérisé en ce que** les lignes électriques passent dans les bras pivotants (18, 19) dans des logements - conçus en conséquence sur la surface intérieure des leviers pivotants respectifs - et quand les bras pivotants (18, 19) sont ouverts, le moteur s'arrête automatiquement.

23. Purificateur d'air selon l'une des revendications 1 à 22, **caractérisé en ce qu'**il est utilisable en particulier comme aspirateur ponctuel, comme appareil de ventilation ou de surpression ou comme nettoyeur à air pur.

24. Purificateur d'air selon la revendication 1 ou 23, **caractérisé en ce que** le purificateur utilisable comme aspirateur ponctuel est pourvu d'un raccordement d'aspiration ponctuel (52) disposé sur un côté du socle (2) de l'enveloppe (1) pour le raccordement d'un tuyau d'aspiration (53).

25. Purificateur d'air selon la revendication 1 ou 23, **caractérisé en ce que** le purificateur d'air utilisable comme appareil de ventilation ou de surpression est pourvu d'un conduit (56) qui est raccordé au raccordement d'aspiration d'appareil et qui est traversé par l'air dans le sens latéral et/ou du bas jusque dans le socle.

26. Purificateur d'air selon la revendication 1 ou 23, **caractérisé en ce que** le purificateur d'air utilisable comme nettoyeur à air pur est pourvu d'un élément d'évacuation (62) qui est conçu comme un cône, un tuyau ou un conduit ou un élément similaire, et qui est traversé par l'air vers l'extérieur.

27. Purificateur d'air selon la revendication 1 ou 23, **caractérisé en ce que** grâce au pivotement des leviers pivotants (18, 19) latéralement à l'opposé de l'enveloppe, un mouvement de soulèvement des modules individuels les uns par rapport aux autres a lieu en supplément à l'opposé du socle (2).
